(19) **Europäisches Patentamt**
**European Patent Office**
**Office européen des brevets**

(11) **EP 2 119 370 A2**

(12) **EUROPEAN PATENT APPLICATION**

(43) Date of publication:
**18.11.2009 Bulletin 2009/47**

(51) Int Cl.:
***A23G 9/32*** (2006.01)     ***A23G 9/48*** (2006.01)
***A23G 1/32*** (2006.01)

(21) Application number: **09158324.5**

(22) Date of filing: **21.04.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **14.05.2008 EP 08156161**

(71) Applicants:
• **Unilever PLC**
**100 Victoria Embankment**
**London, Greater London EC4Y 0DY (GB)**
Designated Contracting States:
**CY GB IE MT**
• **Unilever N.V.**
**3013 AL Rotterdam (NL)**
Designated Contracting States:
**AT BE BG CH CZ DE DK EE ES FI FR GR HR HU IS IT LI LT LU LV MC MK NL NO PL PT RO SE SI SK TR**

(72) Inventors:
• **Berry, Mark, John**
**Bedford, Bedfordshire MK44 1LQ (GB)**
• **Hoddle, Andrew**
**Bedford, Bedfordshire MK44 1LQ (GB)**
• **Nicol, Regina, Beate, Gisela**
**Bedford, Bedfordshire MK44 1LQ (GB)**
• **Pleasants, Michael, William**
**Bedford, Bedfordshire MK44 1LQ (GB)**
• **Velikov, Krassimir, Petkov**
**3133 AT, Vlaardingen (NL)**

(74) Representative: **Clarke, Christopher John**
**Unilever Patent Group**
**Colworth House**
**Sharnbrook**
**Bedford MK11 1LQ (GB)**

(54) **Frozen confectionery product**

(57) A frozen confection product that has a relaxatory effect when consumed is provided, the product comprising a frozen composition and a chocolate composition or a chocolate analogue composition which comprises non-fat cocoa solids. The product comprises at least 0.01 wt% γ-aminobutyric acid (GABA) and at most 0.05 wt% theobromine.

EP 2 119 370 A2

**Description**

**Technical Field of the Invention**

The present invention relates to frozen confectionery products, in particular to frozen confectionery products that have a relaxatory effect when consumed.

**Background to the invention**

Chocolate and chocolate products are believed to be mood enhancing. Part of the reason may be the pleasant taste that can help to make consumers feel happy. Additionally, chocolate contains substances that, when consumed in sufficient quantity, are psycho-pharmacologically active (Smit et al., Psychopharmacology 2004, 176, pp 412-419). These substances include theobromine, caffeine and γ-aminobutyric acid (GABA). Theobromine and caffeine are generally considered to be stimulants.

It would be appealing to many consumers to eat chocolate to enhance their mood rather than, for example, to take prescription medicines. However, the levels of psycho-active substances in chocolate are usually too low to have a substantial effect on mood states. Typically, cocoa mass comprises approximately 1 wt% theobromine, 0.05% GABA and 0.1% caffeine.

JP 2005 / 348656 discloses a food or beverage, in particular chocolate or cocoa, having a relaxatory effect. The product contains elevated levels of GABA. Nonetheless, there remains a need for improved food products with moodenhancing properties, and in particular improved relaxation effects.

**Brief Description of the Invention**

Ice cream has been shown to have an effect on the orbitofrontal cortex, a part of the brain that is known to activate when people enjoy themselves (see for example "The Guardian", April 29 2005). The combination of chocolate and ice cream is therefore an especially suitable means for providing a relaxing food product. Accordingly, in a first aspect, the present invention provides a frozen confection product comprising a frozen composition and a chocolate composition or a chocolate analogue composition, wherein the chocolate or chocoalate analogue composition comprises non-fat cocoa solids, **characterised in that** the product comprises at least 0.01 wt% γ-aminobutyric acid (GABA) and at most 0.05 wt% theobromine.

Preferably the chocolate composition or chocolate analogue composition comprises at least 2.0 wt% non-fat cocoa solids, more preferably at least 5.0 wt%, even more preferably at least 10.0 or 15.0 wt%, most preferably at least 20.0 wt% non-fat cocoa solids.

Preferably the product comprises at least 0.02, more preferably at least 0.05, most preferably at least 0.1wt% GABA.

Preferably the product comprises at most 2.0, more preferably at most 1.0, most preferably at most 0.5 wt% GABA.

Preferably the product comprises at most 0.01 wt% theobromine, more preferably at most 0.005 wt%, most preferably less than 0.001wt%.

Preferably the product comprises at most 0.01 wt% caffeine.

**Detailed Description of the Invention**

All percentages, unless otherwise stated, refer to the percentage by weight, with the exception of percentages cited in relation to the overrun.

<u>Frozen composition</u>

The frozen composition is preferably ice cream, sherbet, sorbet, frozen yoghurt or water ice. Ice cream typically contains fat, non-fat milk solids (of which about one third is milk protein and about half is lactose) and sugars, together with other minor ingredients such as stabilisers, emulsifiers, colours and flavourings. Water ice typically contains sugars together with stabilisers, colours and flavourings.

Frozen compositions of the invention may comprise fat. In a preferred embodiment of the invention, the frozen composition has a fat content of at least 2%, preferably at least 4%, more preferably at least 7%; and at most 20%, preferably at most 15%, more preferably at most 12%. Suitable fats include, but are not limited to dairy fat, coconut oil, palm oil and sunflower oil.

Frozen compositions of the invention may also comprise protein, preferably milk protein. Suitable sources of milk protein include milk, concentrated milk, milk powders, whey, whey powders and whey protein concentrates/isolates. In order to aid in emulsification and/or aeration during manufacture of the frozen composition it is preferable that the protein content is greater than 3% by weight of the frozen composition. In order to prevent the texture of the composition from becoming chalky, it is also preferable that the protein content is less than 8% by weight of the frozen composition. Furthermore, milk is generally believed to aid relaxation. For example, the habit of consuming a milky drink late in the evening is believed to aid sleep. There are compounds in milk that could account for this effect, notably alpha-lactalbumin. Alpha-lactalbumin is a protein that is especially rich in tryptophan, an essential amino acid (i.e. it cannot be synthesised by humans and is therefore needed in the diet). Tryptophan functions as a biochemical precursor for serotonin (a neurotransmitter). In turn, serotonin plays a role in the modulation of mood and sleep. Additionally, the hormone melatonin is present in milk. Melatonin regulates the circadian cycle (the 24 hour "biological clock").

Frozen compositions of the invention may also comprise an emulsifier, such as mono- and di-glycerides of saturated or unsaturated fatty acids, lecithin and egg yolk. The frozen compositions may also comprise a stabiliser, such as locust bean gum, guar gum, agar, alginates, carrageenan, pectin, carboxymethyl cellulose, microcrystalline cellulose, dextran and xanthan. Preferably the emulsifier and stabiliser are each present at a level of 0.05 to 1% by weight of the frozen composition.

In addition, the frozen composition may contain flavouring and/or colouring. Pieces of nut, ginger, biscuit, fruit, fruit puree and the like may also be included.

The frozen composition may be aerated or unaerated. By unaerated is meant an overrun of less then 20%, preferably less than 10%. An unaerated frozen composition is not subjected to deliberate steps such as whipping to increase the gas content. Nonetheless, it will be appreciated that during the preparation of unaerated frozen compositions, low levels of gas, such as air, may be incorporated in the product.

Aerated frozen compositions have an overrun of more than 20%, preferably more than 50%, more preferably more than 75%. Preferably the frozen composition has an overrun of less than 200%, more preferably less than 150%, most preferably less than 120%. Overrun is defined by the following equation and is measured at atmospheric pressure

$$\text{overrun \%} = \frac{\text{density of mix - density of frozen confection}}{\text{density of frozen confection}} \times 100$$

Chocolate / chocolate analogue compositions

The term "chocolate" as used herein includes dark chocolate and milk chocolate. The term "chocolate analogue" means chocolate-like fat-based confectionery compositions made with fats other than cocoa butter (for example cocoa butter equivalents, coconut oil or other vegetable oils). Such chocolate analogues are sometimes known as "couvertures". Chocolate analogues need not conform to standardized definitions of chocolate which are used in many countries.

Chocolate and chocolate analogues used in the present invention contain non-fat cocoa solids (e.g. from cocoa powder, cocoa mass etc.). Chocolate most commonly comes in dark and milk varieties, with the non-fat cocoa solids contributing to the brown coloration. Adults generally prefer milk or dark chocolate; indeed chocolate products which contain very high levels of cocoa solids are increasingly popular with consumers. White chocolate, which does not contain non-fat cocoa solids, is outside the scope of the present invention.

Non-fat cocoa solids contain theobromine and caffeine. Hence chocolates / chocolate analogues that contain non-fat cocoa solids also contain theobromine and caffeine. Although according to JP 2005/348656 the theobromine is said to provide a synergistic relaxation effect when present in combination with the GABA, no evidence of this is provided. In fact, the relaxing effect of the GABA could be cancelled out by the stimulatory effect of the theobromine. Without wishing to be limited by theory, the present inventors believe that to optimise the relaxatory effect, a product should in fact be high in GABA and low in theobromine, and preferably also low in caffeine. Theobromine-free and caffeine-free chocolates and chocolate analogues may be obtained by using a source of non-fat cocoa solids from which the theobromine and / or caffeine has been removed. A process for de-theobrominating cocoa products is described in GB 2 185 376.

In organisms, GABA is synthesized by the decarboxylation of L-glutamic acid catalysed by the enzyme glutamate decarboxylase. GABA is commercially available as a nutritional supplement from many suppliers. Chocolate compositions which contain GABA can be produced by simply mixing GABA into the composition (e.g. into molten chocolate). Preferably, the chocolate or chocolate analogue contains at least 75%, more preferably at least 90% of the GABA which is present in the product.

The chocolate composition or chocolate analogue composition may also contain milk solids, sugar or other sweeteners and flavourings. In one embodiment, the chocolate composition or chocolate analogue composition contains non-fat milk solids in an amount of at least 5 wt%, preferably at least 10 wt%.

The chocolate composition or chocolate analogue composition may be in any suitable form, such as a coating on the frozen composition, as pieces (inclusions) located within the frozen composition, a sauce, e.g. as a ripple or swirl in the frozen composition or simply in the form of a flavouring mixed into the frozen composition.

The present invention will now be further described with reference to the following non-limiting examples.

Example 1: Removal of theobromine and caffeine from cocoa powder

Cocoa powder containing reduced amounts of theobromine and caffeine was prepared as follows.

Firstly, the fat content was determined by removing the fat from a standard cocoa powder (which typically contains 15% fat) by extracting with petroleum spirit. This is because the determination of theobromine and caffeine by HPLC must be carried out on fully defatted cocoa powder. Petroleum spirit extraction does not remove theobromine or caffeine. The amounts of theobromine and caffeine in the defatted cocoa powder were determined by HPLC analysis to be 22 mg/g and 1.5 mg/g respectively.

Theobromine and caffeine were then removed from another sample from the same batch of cocoa powder by Soxhlet extraction with ethanol. A 500 ml round bottom flask was charged with approximately 250-300 ml of ethanol (96% Analar grade, BDH) and placed in a thermostatic isomantle heater inside a fume cupboard. A Soxhlet extractor (Quickfit EX5/55 34/35) was connected directly onto the round bottom flask and a condenser (Quickfit 34/35 300mm Coil Condenser) was connected to the top of the extractor. The apparatus was clamped in place and the condenser was connected to the cold water supply.

A 50ml glass beaker was placed onto on a 4 decimal place analytical balance and zeroed. Two clean filter papers (Whatman No1, 110mm diameter) and a clean extraction thimble (Whatman single thickness cellulose extraction thimble 25mm x 100mm) were placed into the glass beaker and the mass was recorded (i.e. thimble + papers). Approximately 8.0g of the cocoa powder (15% fat) was weighed into the extraction thimble and the total mass of the thimble, filter papers and powder was recorded. To ensure that the cocoa powder could not be washed out of the top of the thimble during extraction, the two filter papers were folded in half 3 times (separately) to produce a planer triangular shape. By opening one of the internal folds a cone shaped plug was produced. This was inserted into the thimble to seal the powder in the extraction tube. This was repeated with the second filter paper. The condenser was removed from the Soxhlet apparatus and the sealed extraction thimble placed into the extraction chamber. The apparatus was then resealed and the isomantle was switched on to full. Once the ethanol started to boil and the condensate was refluxing into the Soxhlet extraction chamber, the power setting was reduced until thermostatic control was gained. The system was checked to ensure that there were no leaks from the connection joints then the apparatus was insulated with aluminium foil around the top half of the round bottom flask to help speed up the reflux process. The extraction was allowed to proceed for 7 hours, after which the isomantle was switched off, the insulation removed and the extractor left to cool down for approximately 30 minutes.

Once cooled, the condenser was removed from the extractor and the thimble recovered using tweezers and placed into a clean 50ml beaker. The extracted thimble was held overnight in the fume cupboard to allow the majority of the ethanol to evaporate. After the extraction thimble had dried overnight the beaker and thimble was placed into a vacuum oven and dried for 3 hours at 40°C and under a vacuum. Once dried, the thimble was placed into a desiccator to cool to room temperature before final weighing. After weighing, the filter papers were removed and the contents of the thimble transferred to a clean mortar and ground to a fine powder with a pestle.

The ethanol extraction removes the fat as well as the theobromine and caffeine. The amount of fat removed was determined by weighing the sample before and after extraction in order to calculate the relative amount of sample used when carrying out the analysis. (The fat constitutes approximately 85% of the material removed by ethanol extraction).

Analysis of the theobromine and caffeine contents was carried out by HPLC as before. The theobromine level was found to be 4.89 mg/g (i.e. 22% of the original value); and the caffeine level was found to be less than 0.1 mg/g (i.e. less than 7% of the original value).

Example 2

A white chocolate suitable for coating ice creams was prepared according to the formulation shown in Table 1. The chocolate was heated to around 45°C.

Table 1

| Ingredient | Amount (wt%) |
|---|---|
| Sucrose | 42 |
| Cocoa butter | 35 |
| Whole milk powder | 22 |
| Emulsifiers | 1.0 |
| Vanillin | 0.1 |
| GABA (obtained from Sigma) | 0.39 |

De-theobrominated, de-fatted cocoa powder (prepared in example 1) was mixed into the molten chocolate in two ratios (2% and 10%) to produce two different brown chocolates. The compositions and GABA / theobromine / caffeine contents are given in Table 2.

Table 2

| Ingredient (wt%) | Chocolate A | Chocolate B |
|---|---|---|
| White chocolate (from Table 1) | 90 | 98 |
| Cocoa powder (from Example 1) | 10 | 2 |

Coated ice cream stick products were produced using each of these chocolates as follows. The ice cream (at a temperature of -18°C) was held by the stick and dipped into the molten chocolate (at 45°C) to form a coating. The ratio of coating to ice cream was 12.5g coating on 34.5g of ice cream in each case. Product A contained 6.1 mg theobromine and 44 mg GABA in 47g of product. Product B contained 1.2 mg theobromine and 48 mg GABA in 47g of product. These amounts are expressed as weight percentages (based on the product) in Table 3.

Table 3

| Amount (wt%) | Product A | Product B |
|---|---|---|
| GABA | 0.094 | 0.10 |
| Theobromine | 0.013 | 0.0026 |
| Caffeine | < 0.0003 | < 0.00006 |

Further products were produced by solidifying the chocolates, breaking them into small pieces and mixing them into ice cream as inclusions at a ratio of 25g inclusions in 69g ice cream.

Example 3

In order to reduce the theobromine and caffeine content of cocoa powder even further, the heated extraction described in example 1 was carried out for a further two seven hour periods, i.e. a total extraction time of 21 hours. The amounts of caffeine and theobromine were determined as before. The theobromine content was found to be 0.9 mg/g (i.e. less than 3% of the original starting level of the standard cocoa powder) and the caffeine content was 0.039 mg/g (i.e. less than 4% of the original starting level of the standard cocoa powder).

Example 4

Chocolates were made using the cocoa powder from example 3 according to the recipes in Table 4. Chocolate C is a milk chocolate, whereas D, E and F are plain (dark) chocolates, containing 60%, 65% and 80% cocoa solids (i.e. non-fat cocoa solids + cocoa butter) respectively.

Table 4

| Ingredient (wt %) | C | D | E | F |
|---|---|---|---|---|
| Cocoa powder (from Example 3) | 3 | 15 | 20 | 35 |
| Sugar | 36.5 | 39.5 | 34.5 | 19.5 |
| Cocoa butter | 40 | 45 | 45 | 45 |
| Lecithin | 0.3 | 0.3 | 0.3 | 0.3 |
| Skim milk powder | 15 | - | - | - |
| Butterfat | 5 | - | - | - |
| GABA (obtained from Fagron B.V.) | 0.2 | 0.2 | 0.2 | 0.2 |

Coated ice cream products were produced as described in example 2 using each of these chocolates. Small ice cream lollies, each weighing 20g were coated with 8g of chocolates C, D and E. An ice cream lolly of 18g was coated with 8g of chocolate F. The amounts of GABA, theobromine and caffeine in these products (expressed as weight percentages based on the product) are given in Table 5.

Table 5

| Ingredient (wt %) | A | B | C | D |
|---|---|---|---|---|
| GABA | 0.058 | 0.058 | 0.058 | 0.062 |
| Theobromine | 0.00077 | 0.0040 | 0.0052 | 0.00098 |
| Caffeine | 0.000033 | 0.00017 | 0.00022 | 0.00042 |

The various features and embodiments of the present invention, referred to in individual sections above apply, as appropriate, to other sections, mutatis mutandis. Consequently features specified in one section may be combined with features specified in other sections, as appropriate. Although the invention has been described in connection with specific preferred embodiments, it should be understood that the invention as claimed should not be unduly limited to such specific embodiments. Indeed, various modifications of the described modes for carrying out the invention which are apparent to those skilled in the relevant fields are intended to be within the scope of the following claims.

Claims

1. A frozen confection product comprising a frozen composition and a chocolate composition or a chocolate analogue composition, wherein the chocolate or chocolate analogue composition comprises non-fat cocoa solids, **characterised in that** the product comprises at least 0.01 wt% γ-aminobutyric acid (GABA) and at most 0.05 wt% theobromine.

2. A frozen confection product according to claim 1 wherein the chocolate composition or chocolate analogue composition comprises at least 2.0 wt% non-fat cocoa solids.

3. A frozen confection product according to claim 1 wherein the chocolate composition or chocolate analogue composition comprises at least 5.0 wt% non-fat cocoa solids.

4. A frozen confection product according to claim 1 wherein the chocolate composition or chocolate analogue composition comprises at least 10.0 wt% non-fat cocoa solids.

5. A frozen confection product according to claim 1 wherein the chocolate composition or chocolate analogue composition comprises at least 15.0 wt% non-fat cocoa solids.

6. A frozen confection product according to claim 1 wherein the chocolate composition or chocolate analogue composition comprises at least 20.0 wt% non-fat cocoa solids.

**7.** A frozen confection product according to any of claims 1 to 6 which comprises from 0.02 to 2.0 wt% GABA.

**8.** A frozen confection product according to any of claims 1 to 6 which comprises at most 0.01 wt% theobromine.

**9.** A frozen confection product according to claim 8 which comprises less than 0.001 wt% theobromine.

**10.** A frozen confection product according to any of claims 1 to 9 which comprises at most 0.01 wt% caffeine.

**11.** A frozen confection product according to any of claims 1 to 10 wherein at least 75% of the GABA is present in the chocolate composition or chocolate analogue composition.

**12.** A frozen confection product according to any of claims 1 to 11 wherein the chocolate composition or chocolate analogue composition comprises at least 5 wt% non-fat milk solids.

**13.** A frozen confection product according to any of claims 1 to 12 wherein the chocolate composition or a chocolate analogue composition is in the form of a coating on the frozen composition and / or inclusions located within the frozen composition and / or a ripple or swirl of sauce in the frozen composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005348656 A

- GB 2185376 A

**Non-patent literature cited in the description**

- **Smit et al.** *Psychopharmacology,* 2004, vol. 176, 412-419

- *The Guardian,* 29 April 2005